# FASCICULE DE BREVET EUROPEEN

(11) **EP 0 542 655 B1**
(45) Date de publication et mention de la délivrance du brevet: **03.07.1996**
(21) Numéro de dépôt: 92440126.8
(22) Date de dépôt: 12.11.1992
(51) Int. Cl.: G01N 33/555, B01L 3/14

(54) **Dispositif permettant de réaliser en un seul temps la séparation et la mise en évidence des agglutinats érythrocytaires**
Vorrichtung zur Trennung und Bestimmung der Erythrozyt-Agglutinate in einem Schritt
Device for the separation and determination of agglutinated erythrocytes in a single step

(30) Priorité: 12.11.1991 FR 9114061
(43) Date de publication de la demande: 19.05.1993
(73) Titulaire: INSTITUT JACQUES BOY, F-51000 Reims (FR)
(72) Inventeur: Majurel, Marc, F-08190 Poilcourt Sydney (FR)
(74) Mandataire: Mayran, Ninon, Avocat

(56) Documents cités:
- EP-A- 0 131 934
- EP-A- 0 269 240
- EP-A- 0 325 910
- US-A- 4 933 092
- DATABASE WPIL, week 8724, Derwent Publications Ltd., London (GB); AN 87-166227/

## Description

La présente invention a pour objet un dispositif permettant en un seul temps la séparation d'agglutinats érythrocytaires des hématies libres lors d'un test d'agglutination, et la mise en évidence desdits agglutinats.

On sait que, en immuno-hématologie érythrocytaire, la réaction antigène-anticorps peut être mise en évidence grâce à la présence d'agglutinats. Cette agglutination, spontanée en milieu salin, n'est toutefois pas toujours observable in vitro, notamment dans le cas où les anticorps sont des anticorps dits sensibilisants, les hématies sensibilisées ne donnant pas lieu à une agglutination.

Afin de provoquer l'agglutination des hématies sensibilisées, diverses techniques ont été mises au point, parmi lesquelles les plus couramment utilisées consistent à ajouter au milieu réactionnel :
- soit des macromolécules comme l'albumine, le dextran ou la polyvinylpyrrolidone;
- soit, après lavage des hématies sensibilisées, une antiglobuline humaine (test de Coombs) ;
- soit une molécule polycationique comme le bromure d'hexadiméthrine, ou "polybrène", le traitement de l'agglutinat ainsi obtenu par une solution citratée provoquant la remise en suspension des hématies non sensibilisées (technique de Lalezari).

Une autre technique consiste à traiter préalablement les hématies par une enzyme protéolytique comme la papaïne, la broméline ou la trypsine.

Toutes ces techniques d'agglutination peuvent être mises en oeuvre manuellement ou automatiquement, dans les supports les plus variés : tubes à hémolyse, plaques de verre, rhésuscope, microplaques à 96 puits.

Toutefois, elles font généralement appel à une centrifugation et tant les phases de lavage (pour le test de Coombs) que les temps et vitesses de centrifugation ainsi que le type d'agitation pour la remise en suspension finale constituent des paramètres critiques qui rendent la maîtrise de ces réactions délicate.

D'autre part, les dispositifs mis en oeuvre dans ces techniques de séparation et de mise en évidence des agglutinats érythrocytaires sont peu nombreux.

Graham et al. a proposé en 1982 un tube capillaire spécifique que l'on remplit d'une solution d'albumine et de dextran, de densité intermédiaire entre sérum et globules, toutefois cette technique ne s'est pas révélée plus sensible que le test de Coombs classique.

Une autre technique dite "en phase solide", a été présentée en 1984 au symposium de Munich par une équipe française, MULLER A. et al., consistant à centrifuger le mélange sérum-globules à travers un liquide de séparation, après que les puits des microplaques utilisées aient été revêtus d'antiglobuline, la lecture étant faite automatiquement.

Le brevet français 2 577 321 décrit une méthode dans laquelle le milieu de séparation est constitué d'un gel à base de dextran, toutefois cette méthode, comme la plupart des méthodes antérieurement proposées, fait appel à une centrifugation, avec l'inconvénient inhérent à la mise en oeuvre de ce type de technique, à savoir de nécessiter un matériel qui ne peut être transporté hors du laboratoire.

La présente invention a pour but de remédier à ces divers inconvénients des techniques connues en proposant un dispositif qui permet en un seul temps la séparation et la mise en évidence des agglutinats érythrocytaires, en évitant le recours à une centrifugation.

La présente invention a ainsi pour objet un dispositif permettant la séparation et la mise en évidence des agglutinats érythrocytaires, ledit dispositif présentant la caractéristique essentielle de comprendre au moins un tube capillaire ouvert à son extrémité inférieure, laquelle est munie d'un filtre constitué d'une membrane composite surmontée d'une couche de microbilles de verre de 2 à 3 mm d'épaisseur.

Selon une autre caractéristique du dispositif selon l'invention, les microbilles de verre mises en oeuvre présentent un diamètre moyen compris entre 20 et 50 µm, tandis que la membrane composite qui obture le tube capillaire à son extrémité inférieure présente une ouverture de mailles de 5 à 7 µm, autorisant le passage des seuls globules rouges non agglutinés.

Selon une caractéristique additionnelle du dispositif selon l'invention, la membrane composite mise en oeuvre pour obturer le tube capillaire à son extrémité inférieure est constituée des trois couches ci-après :
- une première couche très hydrophile au contact des microbilles.
- une couche intermédiaire modérément hydrophobe
- une couche externe fortement hydrophobe percée en son centre d'un orifice d'environ 0,1mm de diamètre pour l'écoulement du flux de la phase mobile.

La première couche de la membrane composite du dispositif selon l'invention est constituée d'un matériau très hydrophile tel qu'un polyamide 66, présentant une ouverture de mailles de 5 à 7 µm. Cette première couche détermine la porosité de la membrane composite.

La couche intermédiaire de ladite membrane composite est constituée d'un matériau fibreux modérément hydrophobe, tel qu'un polypropylène, qui empêche l'écoulement du liquide pendant la phase d'incubation et assure d'autre part une fonction de drainage de la phase mobile pendant l'écoulement.

La couche externe de la membrane composite du dispositif selon l'invention est constituée d'un matériau fortement hydrophobe, comme un polymère de tétrafluoroéthylène.

Selon un premier mode de réalisation de l'invention, le dispositif comprend un tube capillaire d'environ 60 mm de hauteur et d'un diamètre interne d'environ 2 mm, réalisé en un matériau approprié, tel que le verre ou le polystyrène cristal.

Dans ce cas la membrane composite peut être fixée au tube à l'aide d'un manchon semi-rigide glissé autour du tube, comme un manchon en chlorure de polyvinyle.

Selon un second mode de réalisation de l'invention, le dispositif comprend une microplaque à 96 puits, munie à sa base d'une membrane composite telle que décrite ci-dessus. Dans ce cas la membrane est solidarisée par thermosoudage à la périphérie de chacun des puits.

Les réactifs nécessaires au test d'agglutination peuvent être disposés préalablement à la surface des billes ou ajoutés extemporanément avec les hématies ou les sérums à tester, ces réactifs pouvant être n'importe quel réactif mis en oeuvre à cet effet dans les techniques classiques.

Lorsque la phase d'incubation est achevée, le contenu du tube est soumis, à sa partie inférieure, à l'action d'un moyen permettant de déplacer la phase mobile, ledit moyen pouvant consister soit en un absorbant constitué de matière cellulosique à haut pouvoir de rétention d'eau, mis en place au contact du filtre, soit en une dépression créée dans une chambre mise au contact du filtre. Ce dernier moyen est préférentiellement mis en oeuvre lorsque la technique est automatisée.

Le dispositif selon l'invention permet d'effectuer la lecture du résultat à la surface des billes, une faible épaisseur de billes étant suffisante pour séparer les hématies libres des hématies liées. Une lecture par le dessus de l'ensemble des puits d'une microplaque est ainsi rendue possible tout en conservant une bonne sensibilité.

Le dispositif selon l'invention présente l'avantage d'être parfaitement autonome, ne nécessitant aucun matériel annexe tel qu'une centrifugeuse, en sorte qu'il peut être aisément mis en oeuvre hors du laboratoire.

Il présente également l'avantage d'offrir un champ d'application large, pouvant être utilisé aussi bien en sérologie (hémagglutination) que pour des groupages sanguins ou des recherches d'agglutinines irrégulières.

Il peut en outre être utilisé pour la mise en oeuvre de toutes les techniques connues d'agglutination, et il peut être aisément automatisé, notamment dans le cas où il est mis en oeuvre dans des microplaques 96 puits.

A ces avantages s'ajoute celui inhérent à la stabilité de l'image produite, les réactions restant visibles plusieurs heures.

Le procédé selon l'invention permet aussi d'éviter les phases critiques et longues que constituent les lavages et la centrifugation, tout en conservant une grande sensibilité.

D'autre part, le ou les tubes mis en oeuvre étant ouverts, le procédé selon l'invention permet de cumuler deux techniques connues en associant les deux réactifs correspondants, introduits successivement : on peut ainsi réaliser une technique mixte, par exemple en mettant en oeuvre le polybrène et une antiglobuline humaine.

Enfin, la mise en oeuvre de microbilles de verre permet de réaliser des dispositifs prêts à l'emploi, par exemple sous forme de microplaques à 96 puits dont chaque puits renferme une couche desdites microbilles, ce qui est impossible avec des microbilles de polymères, qui se rétractent en séchant.

Les exemples qui suivent sont fournis à titre d'illustration de la présente invention, vis-à-vis de laquelle ils ne présentent aucun caractère limitatif.

### EXEMPLE 1 : réalisation d'une microplaque prête à l'emploi.

On réalise une microplaque à 96 puits selon l'invention en en obturant le fond à l'aide d'une membrane composite fixée par thermosoudage à la périphérie de chaque puits, et constituée des trois couches ci-après :
- polyamide 66 de porosité 5µm
- polypropylène
- polymère de tétrafluoroéthylène (PTFE),
cette dernière couche étant percée au centre de chaque puits d'un orifice d'environ 0,1mm.

On dépose dans chaque puits 200 µl d'un mélange volume à volume d'eau et de billes de verre de diamètre 37 à 50 µm.

Après aspiration de l'eau à travers les orifices de la membrane, on sèche à l'étuve à 37 °C pendant une heure.

On obtient ainsi une plaque prête à l'emploi qui se conserve à température ambiante sans limite de temps.

### EXEMPLE 2 : Groupage ABO et Rhésus Standard

Dans des puits adjacents d'une microplaque préparée selon l'exemple 1, on peut réaliser le groupage ABO et Rhésus Standard selon les techniques de Beth-Vincent et Simonin, simultanément.

### 1° Technique Beth-Vincent (Recherche des antigènes de type A ou/et B)

On introduit successivement :
- 50 µl d'hématies à tester, mises à 5 % dans une solution de broméline à 0,25 %
- 50 µl de sérums tests dilués anti A ou anti B ou anti AB.

On laisse incuber cinq minutes à 4° C puis on aspire sous vide de 10 cm Hg et l'on rince avec 500 µl de détergent "BRIJ® 56" à 0,075 %.

La lecture du résultat s'opère aisément à la surface des billes de verre.

### 2° Technique Simonin (Recherche des anticorps de type A ou/et B)

On introduit successivement :
- 50 µl d'hématies tests, mises à 5 % dans une solution de broméline à 0,25 %
- 100 µl de sérum à tester

On laisse incuber cinq minutes à 4° C puis on aspire sous vide de 10 cm Hg et l'on rince avec 250 µl de "BRIJ® 56" à 0,075 %

La lecture du résultat s'opère aisément à la surface des billes de verre.

### 3° Détermination du Rhésus Standard (Recherche des antigènes D)

On introduit successivement :
- 50 µl d'hématies à tester à 5 % dans une solution de "BRIJ® 56" à 0,075 %
- 100 µl de sérum test anti D dilué

On laisse incuber cinq minutes à 4° C puis on aspire sous vide de 10 cm Hg et l'on rince avec 500 µl de "BRIJ® 56" à 0,075 %.

La lecture du résultat s'opère aisément à la surface des billes de verre.

Lecture du résultat pour toutes les techniques utilisées :
- résultat positif : hématies agglutinées formant un tapis rouge à la surface des billes de verre
- résultat négatif : pas d'hématies visibles à la surface des billes de verre.

### EXEMPLE 3

Au fond de chaque puits d'une microplaque préparée selon l'exemple 1 on dépose successivement :
- Hématies tests à 3 % - 1 vol. (50 µl)
- Sérum à tester - 1 vol. (50 µl)
- Polybrène à 1 % - 1 vol. (50 µl)

On laisse incuber dix minutes à température ambiante, puis on aspire sous vide de 10 cm Hg.

On obtient des tapis cellulaires uniformes dans tous les puits, sur lesquels on ajoute 10 µl d'antiglobuline polyvalente, puis on remplit la totalité des puits avec une solution de lavage (citrate hypertonique) additionnée de TW20 à 1 % (U/U).

Après écoulement total de la solution de lavage, on procède à la lecture, en observant la partie supérieure de la couche de billes de verre.

## Revendications

1. Dispositif permettant de réaliser en un seul temps la séparation et la mise en évidence des agglutinats érythrocytaires lors des tests d'agglutination, caractérisé en ce qu'il comprend au moins un tube capillaire ouvert à son extrémité inférieure, laquelle est munie d'un filtre constitué d'une membrane composite surmontée d'une couche de microbilles de verre de diamètre moyen compris entre 20 et 50 µm, ladite membrane composite comprenant les trois couches successives suivantes :
- une première couche très hydrophile, de porosité 5 à 7 µm, au contact des microbilles de verre.
- une seconde couche, intermédiaire, modérément hydrophobe.
- une troisième couche, externe, fortement hydrophobe, percée en son centre d'un orifice d'environ 0,1 mm de diamètre.

2. Dispositif selon la revendication 1, caractérisé en ce que la couche de microbilles de verre a une épaisseur de 2 à 3 mm.

3. Dispositif selon la revendication 1, caractérisé en ce que la première couche de la membrane composite est réalisée en polyamide 66 très hydrophile.

4. Dispositif selon l'une quelconque des revendications précédentes, caractérisé en ce que la deuxième couche de la membrane composite est réalisée en un matériau fibreux comme le polypropylène.

5. Dispositif selon l'une quelconque des revendications précédentes, caractérisé en ce que la troisième couche de la membrane composite est réalisée en un polymère de tétrafluoroéthylène (PTFE).

6. Dispositif selon l'une quelconque des revendications 1 à 5, caractérisé en ce qu'il comprend un tube d'environ 60 mm de hauteur et d'environ 2 mm de diamètre interne, à l'extrémité inférieure duquel la membrane composite est fixée par tout moyen approprié.

7. Dispositif selon la revendication 6, caractérisé en ce que la membrane est fixée autour de la partie inférieure du tube à l'aide d'un manchon semi-rigide du type chlorure de polyvinyle.

8. Dispositif selon l'une quelconque des revendications 1 à 5, caractérisé en ce qu'il comprend une microplaque à 96 puits dont l'extrémité inférieure est obturée par la membrane composite, fixée par thermosoudage à la périphérie de chaque puits.

## Patentansprüche

1. Vorrichtung zur Trennung und Bestimmung der Erythrozyt-Agglutinate beim Agglutinationstest in einem Schritt, dadurch gekennzeichnet, daß diese wenigstens ein Kapillarrohr aufweist, das an seinem unteren Ende offen ist, und das versehen ist mit einem Filter, bestehend aus einer zusammengesetzten Membran, überlagert von einer Schicht aus Mikrokugeln von einem mittleren Durchmesser zwischen 20 und 50 µm, wobei die zusammengesetzte Membran die drei folgenden, aufeinanderfolgenden Lagen umfaßt:
eine erste Lage, die sehr hydrophil ist, mit einer Porosität von 5 bis 7 µ, im Kontakt mit den Mikrokugeln aus Glas,
eine zweite Zwischenlage, die mäßig hydrophob ist,
eine dritte, äußere, stark hydrophobe Lage, die in Ihrem Zentrum von einer Öffnung von etwa 0,1 mm Durchmesser durchbohrt ist.

2. Vorrichtung nach Anspruch 1, dadurch gekennzeichnet, daß die Schicht aus Mikrokugeln aus Glas eine Stärke von 2 bis 3 mm hat.

3. Vorrichtung nach Anspruch 1, dadurch gekennzeichnet, daß die erste Lage der zusammengesetzten Membran aus sehr hydrophylem Polyamid (66) besteht.

4. Vorrichtung nach einem der vorausgegangenen Ansprüche, dadurch gekennzeichnet, daß die zweite Lage der zusammengesetzten Membran aus einem faserigen Material wie Polypropylen besteht.

5. Vorrichtung nach einen der vorausgegangenen Ansprüche, dadurch gekennzeichnet, daß die dritte Lage der zusammengesetzten Membran aus einem Tetrafluoroethylenpolymer (PTFE) besteht.

6. Vorrichtung nach einem der Ansprüche 1 bis 5, dadurch gekennzeichnet, daß diese ein Rohr von etwa 60 mm Höhe und ungefähr 2 mm Innendurchmesser aufweist, an dessen unterem Ende die zusammengesetzte Membran durch geeignete Mittel befestigt ist.

7. Vorrichtung nach Anspruch 6, dadurch gekennzeichnet, daß die Membran um den unteren Teil des Rohres mittels einer halbstarren Manschette aus Polyvinylchlorid befestigt ist.

8. Vorrichtung nach einem der Ansprüche 1 bis 5, dadurch gekennzeichnet, daß diese eine Mikroplatte mit 96 Bohrungen aufweist, deren unteres Ende durch die zummmangesetzte Membran abgesperrt ist, fixiert durch Thermoschweißung am Umfang einer jeden Bohrung.

## Claims

1. Device for the separation and determination of agglutinated erythrocytes in a single step during agglutination tests, characterised in that it comprises at least one capillary tube open at its lower end, which is provided with a filter consisting of a composite membrane covered by a layer of glass micro-balls with an average diameter of between 20 and 50 µm, this composite membrane comprising the following three successive layers:
- a first, highly absorbent layer, with porousness of 5 to 7 µm in contact with the glass micro-balls;
- a second, intermediate layer, which is moderately absorbent; and
- a third, outer layer, which is very absorbent, in the centre of which there is an aperture approximately 0.1 mm in diameter.

2. Device according to claim 1, characterised in that the layer of glass micro-balls is 2 to 3 mm thick.

3. Device according to claim 1, characterised in that the first layer of the composite membrane consists of highly absorbent polyamide 66.

4. Device according to one of the preceding claims, characterised in that the second layer of the composite membrane consists of a fibrous material such as polypropylene.

5. Device according to one of the preceding claims, characterised in that the third layer of the composite membrane consists of a tetrafluoroethylene polymer (PTFE).

6. Device according to any one of claims 1 to 5, characterised in that it comprises a tube approximately 60 mm high with an inner diameter of 2 mm, at the lower end of which the composite membrane is attached by any appropriate means.

7. Device according to claim 6, characterised in that the membrane is attached around the lower part of the tube by means of a semi-rigid sleeve of the polyvinylchloride type.

8. Device according to any one of claims 1 to 5, characterised in that it comprises a 96-hole microplate, the lower end of which is sealed by the composite membrane, which is attached by heat sealing to the periphery of each hole.
